# EUROPEAN PATENT APPLICATION

(11) **EP 1 950 566 A1**
(43) Date of publication of application: **30.07.2008**
(21) Application number: 06832483.9
(22) Date of filing: 10.11.2006
(51) Int. Cl.: G01N 33/543, C12Q 1/02, G01N 21/64, G01N 33/48, G01N 33/53

(54) **METHOD OF EVALUATING BINDING PROPERTIES OF BIOSUBSTANCES AND BIOSUBSTANCE-BINDING SUBSTANCES**

(30) Priority: 10.11.2005 JP 2005326543
(71) Applicant: National University Corporation University Of Toyama, Toyama-shi, Toyama 930-8555 (JP)
(72) Inventor: KISHI, Hiroyuki, Toyama-shi Toyama 930-0886 (JP); TAJIRI, Kazuto, Toyama-shi Toyama 930-0138 (JP); MURAGUCHI, Atsushi, Toyama-shi Toyama 930-0001 (JP)
(74) Representative: Patentanwälte Ruff, Wilhelm, Beier, Dauster & Partner
(86) International application number: PCT/JP2006/322431
(87) International publication number: WO 2007/055302

(57) **Abstract**

[PROBLEMS]

To provide a method whereby the binding properties between a biosubstance such as a cell and two or more substances without resorting to a complicated and expensive apparatus.

[MEANS FOR SOLVING PROBLEMS]

A method of evaluating binding properties of multiple biosubstance-binding substances having binding properties to multiple biosubstance against multiple biosubstances immobilized on a substrate surface, comprising:
(a1) bringing a first biosubstance-binding substance labeled with an extinguishable fluorescent substance into contact with said multiple immobilized biosubstances;
(b1) irradiating said multiple immobilized biosubstances with the excitation beam of the extinguishable fluorescent substance;
(c1) detecting the biosubstances that have bound to said biosubstance binding-substance labeled with said extinguishable fluorescent substance based on the fluorescence emitted by said extinguishable fluorescent substance due to said excitation beam,
(d1) extinguishing the extinguishable fluorescent substance that has emitted the fluorescence; then
(a2) bringing a second biosubstance-binding substance labeled with an extinguishable fluorescent substance into contact with said multiple immobilized biosubstances following extinction; thereafter
(b2) irradiating the excitation beam of said extinguishable fluorescent substance;
(c2) detecting the biosubstances that have bound to said biosubstance binding-substance labeled with said extinguishable fluorescent substance based on the fluorescence emitted by said extinguishable fluorescent substance; and
(d2) extinguishing said extinguishable fluorescent substance that has emitted fluorescence.

## Description

### Technical Field

The present invention relates to a method of evaluating the binding properties of biosubstances and biosubstance-binding substances. The method of the present invention is also applicable to methods of screening for substances specifically binding a biosubstance.

### Background Art

Thus far, when analyzing tissues and cells which have been stained with various antibodies, it has been necessary to label the various antibodies with different fluorescent pigments, use the florescence-labeled antibodies to stain the tissues and cells, and employ a device equipped with detectors (such as combinations of photomultipliers and optical filters) capable of detection by identifying the various fluorescent pigments. Such devices are generally expensive and limited in the number of pigments they can analyze. One example of such a device is the flow cytometer *(*Lymphocytic Function Detection Methods, Junichi Yada, Michio Fujiwara, ed., Chugai Pharmaceutical (Tokyo) (Nonpatent Reference 1)).

### Disclosure of Invention

### Problems to Be Solved by the Invention

When analyzing cells with a flow cytometer, the same cell cannot be observed twice because the cells are constantly flowing rapidly through a sheath fluid. Accordingly, the types of fluorescent pigments that can be identified in a flow cytometer are determined by the types of lasers and how many combinations of optical filter and photomultiplier are installed.

Thus, an object of the present invention is to provide a method of evaluating the binding properties of two or more substances to a biosubstance such as single cells without employing a complex, expensive device.

The present inventors conducted various researches into achieving the above-stated object. As a result, they discovered that when samples labeled with phycoerythrin (PE), a fluorescent protein emitting an orange fluorescence that is commonly employed in flow cytometry, were observed with a fluorescence microscope (under a mercury lamp), extinction occurred in several minutes and the fluorescence ended up being eliminated. By actively exploiting this phenomenon, a simple fluorescence-detection device could be used to detect multiple biosubstances with multiple PE-labeled antibodies (biosubstance-binding substances). That is, employing multiple types of antibodies for a PE-labeled cell surface marker protein, the cells were first stained with a PE-labeled antibody 1 and observed with a scanner. Following observation, the PE was extinguished under a fluorescence microscope. Next, a separate PE-labeled antibody 2 was used to stain the cells and they were observed with a scanner. Following observation, the PE was extinguished under a fluorescence microscope. It was discovered that by repeating the staining/observation/extinction cycle in this manner, the cell surface protein expression of a single cell could be analyzed with limitless antibodies. The present invention was devised on that basis.

### Means for solving the problems

The present invention is as follows.
[1] A method of evaluating binding properties of multiple biosubstance-binding substances having binding properties to multiple biosubstances against multiple biosubstances immobilized on a substrate surface, comprising:
   (a1) bringing a first biosubstance-binding substance labeled with an extinguishable fluorescent substance into contact with said multiple immobilized biosubstances;
   (b1) irradiating said multiple immobilized biosubstances with the excitation beam of the extinguishable fluorescent substance;
   (c1) detecting the biosubstances that have bound to said biosubstance binding-substance labeled with said extinguishable fluorescent substance based on the fluorescence emitted by said extinguishable fluorescent substance due to said excitation beam,
   (d1) extinguishing the extinguishable fluorescent substance that has emitted the fluorescence; then
   (a2) bringing a second biosubstance-binding substance labeled with an extinguishable fluorescent substance into contact with said multiple immobilized biosubstances following extinction; thereafter
   (b2) irradiating the excitation beam of said extinguishable fluorescent substance;
   (c2) detecting the biosubstances that have bound to said biosubstance binding-substance labeled with said extinguishable fluorescent substance based on the fluorescence emitted by said extinguishable fluorescent substance; and
   (d2) extinguishing said extinguishable fluorescent substance that has emitted fluorescence.
[2] The method in accordance with [1] wherein following (d2), the steps of (a3) bringing a third or subsequent biosubstance-binding substance labeled with an extinguishable fluorescent substance into contact with said multiple immobilized biosubstances following extinction; (b3) irradiating the excitation beam of said extinguishable fluorescent substance; (c3) detecting the biosubstances that have bound to said biosubstance binding-substance labeled with said extinguishable fluorescent substance based on fluorescence from said extinguishable fluorescent substance; and (d3) extinguishing said extinguishable fluorescent substance that has emitted the fluorescence, are successively repeated one time each for a third and subsequent biosubstance-binding substances.
[3] The method in accordance with [1] or [2] wherein said biosubstance is one or more substances selected from the group consisting of cells, tissues, viruses, bacteria, proteins, nucleic acids, lipids, sugar chains, hormones, and products thereof.
[4] The method in accordance with any one of [1] to [3] wherein said biosubstance-binding substance is one or more members selected from the group consisting of antibodies, antigens, ligands, hormones, proteins, nucleic acid, lipids, and sugar chains.
[5] The method in accordance with any one of [1] to [4] wherein said extinguishable fluorescent substance is phycoerythrin (PE), fluorescein, or allophycocyanine (APC).
[6] The method in accordance with any one of [1] to [5] wherein the florescence of said extinguishable fluorescent substance is extinguished by means of irradiation by a mercury lamp or a halogen lamp.
[7] The method in accordance with any one of [1] to [6] wherein said excitation beam of said extinguishable fluorescent substance is a monochromatic beam.
[8] The method in accordance with [7] wherein said monochromatic beam is a laser beam.
[9] The method in accordance with any one of [1] to [8] wherein data relating to fluorescence in the form of the evaluation results of the binding properties of the biosubstance-binding substances to the multiple substances immobilized on the substrate surface are stored in a computer and a biosubstance having desired binding properties is selected from the stored data.
[10] The method in accordance with [9] wherein the biosubstance that is selected is removed from the substrate surface and employed in a subsequent operation.
[11] The method in accordance with [10], wherein said biosubstances are cells and said method is employed to evaluate the binding properties of cells and cell surface markers.
[12] A method of screening for substances having specific binding properties to biosubstances by employing the method in accordance with any one of [1] to [11].

### Effects of the Invention

1. Analysis of multiple markers is possible in a PE-detecting device even when the device is incapable of detecting other pigments. The analysis device is thus rendered inexpensive.
2. Theoretically, an infinite number of markers can be analyzed. However, in practice, the number of markers that can be analyzed is limited by cell and sample damage and the like.

### Best Mode for Carrying Out the Invention

The method of the present invention is a method of evaluating the binding properties of multiple biosubstance-binding substances having binding properties to multiple biosubstances against multiple biosubstances immobilized on a substrate surface.
The biosubstances employed in the method of the present invention are not specifically limited. Examples are one or more substances selected from the group consisting of cells, tissues, viruses, bacteria, proteins, nucleic acids, lipids, sugar chains, hormones, and their products.

The biosubstances are those immobilized on a substrate surface. The addresses of the cells being observed are fixed so that the binding properties of the multiple biosubstance-binding substances can be readily determined by multiple cycles of excitation, fluorescence detection, and extinction. When cells are constantly moving, as in a flow cytometer, and staining is conducted with multiple antibodies, it is impossible to check whether or not various antibodies bind to the same cell or to different cells. Examples in which the addresses of the biosubstances being observed, such as cells, are immobilized are tissue sections, cell adhesive preparations, and non-adherent cells (lymphocytes) inoculated onto microarray chips.

When the biosubstance is cells, they may be in the form of a microwell array chip prepared by the methods described in Japanese Unexamined Patent Publication (KOKAI) Nos. 2004-173681 and 2004-187676, for example. A further example is a cell chip or protein chip such as that described in Gendai Kagaku, 2002, No. 372, p. 2053-2063.

The biosubstance-binding substance is not specifically limited. Examples are one or more substances selected from the group consisting of antibodies, antigens, ligands, hormones, proteins, nucleic acids, lipids, and sugar chains.

The biosubstance-binding substance employed in the present invention is labeled with an extinguishable fluorescent substance. It suffices for the extinguishable fluorescent substance not to be extinguished by a monochromatic beam (low-energy beam) such as a laser beam, but for it to undergo a change in structure or the like, ceasing to exhibit fluorescence, when irradiated by a high-energy beam from a mercury lamp and the like, or when irradiated for an extended period. Examples of such substances are phycoerythrin (PE), fluorescein (fluorescein isothiocyanate (FITC), for example), or allophycocyanine (APC). One example in addition to PE is PC5. PC5 is obtained by bonding PE and Cy5. Cy5 is excited by the orange fluorescence emitted by PE when it is excited; the fluorescence of Cy5 is detected. PC5 may also be employed as the extinguishable fluorescent substance.

The biosubstance-binding substance labeled with an extinguishable fluorescent substance can be prepared as follows when the biosubstance-binding substance is a protein. In PE labeling of a protein, N-succinimidyl-S-acetylthioacetate (SATA) is first bound to the PE. Next, gamma-maleimidobutyric acid N-hydroxysuccinimide ester (GMBS) is bound to the protein. Finally, the PE and the protein are bound through the SATA and GMBS.
(Reference)
Current Protocols in Immunology, edited by John E. Coligan, Ada M. Kruisbeek, David H. Margulies, Ethan M. Shevach, Warren Strober. Published by Greene Publishing Associated and Wiley-Interscience, New York.

The PC5-labeled biosubstance-binding substance can be prepared using N-succinimidyl 3-(2-pyridylthio)propionate (SPDP) when the biosubstance-binding substance is a protein, for example.
(References)
Annals of the New York Academy of Science, 677: 185-193, 1993; The Journal of Cell Biology, 93: 981-986, 1982.

An FITC-labeled biosubstance-binding substance can be prepared by a method employing FITC on Celite when the biosubstance-binding substance is a protein. Specifically, 1 mg of FITC on Celite 10 percent (Sigma F1628) is added to a (1 mg equivalent) protein solution and the mixture is incubated for 30 minutes at room temperature. The Celite is then removed by centrifugation for 1 minute at 10,000 rpm. Subsequently, the protein solution is dialyzed with PBS to prepare FITC-labeled protein. The FITC itself can be bound to an amino acid in the form of lysine or an N-terminal amino group. Since all proteins have an N terminal, this method permits the labeling of almost any protein.

The method of the present invention comprises:
(a1) bringing a first biosubstance-binding substance labeled with an extinguishable fluorescent substance into contact with multiple immobilized biosubstances;
(b1) irradiating the multiple immobilized biosubstances with the excitation beam of the extinguishable fluorescent substance;
(c1) detecting the biosubstances that have bound to said biosubstance binding-substance labeled with said extinguishable fluorescent substance based on fluorescence emitted by said extinguishable fluorescent substance due to said excitation beam, and
(d1) extinguishing said extinguishable fluorescent substance that has emitted said fluorescence.

A biosubstance binding with the first biosubstance-binding substance can be specified by (a1) to (c1). Following contact between the first biosubstance-binding substance labeled with an extinguishable fluorescent substance with the multiple immobilized biosubstances in (a1), it is suitable to wash the surface of the chip or the like on which the biosubstances are immobilized to remove biosubstance-binding substances in a free state. This washing can be conducted as follows, for example. First, PBS, medium, and other buffers on the surface of the chip or the like on which the biosubstances have been immobilized are removed with a pipet or the like, and new buffer is added. The buffer is removed and a new buffer is added. Repeating this operation several times removes any biosubstance binding-substances present in a free state.

When washing has been completed, the multiple immobilized biosubstances are irradiated with a beam exciting the extinguishable fluorescent substance in (b1) and fluorescence from the biosubstance-binding substance that has bound to a biosubstance is detected in (c1), permitting the specification of a biosubstance exhibiting a binding property to the biosubstance-binding substance.

The first biosubstance-binding substance labeled with an extinguishable fluorescent substance can be contacted with the multiple immobilized biosubstances, for example, by feeding a solution containing the first biosubstance-binding substance labeled with an extinguishable fluorescent substance onto the surface of a chip on which the multiple biosubstances are immobilized. The contact conditions (such as temperature, time, atmosphere, and the presence of additives) can be suitably set based on the type of biosubstance-binding substance and biosubstances.

In (d1), the extinguishable fluorescent substance that has emitted the fluorescence is extinguished. The extinction can be conducted, for example, by irradiation with a mercury lamp. The intensity and duration of illumination by the mercury lamp can be suitably determined taking into account the extinction characteristics of the extinguishable fluorescent substance.

Next, in (a2), a second biosubstance-binding substance labeled with an extinguishable fluorescent substance is brought into contact with the multiple immobilized biosubstances following extinction.
Subsequently, the steps of (b2) irradiating the excitation beam of the extinguishable fluorescent substance; (c2) detecting the biosubstances that have bound to the biosubstance binding-substance labeled with the extinguishable fluorescent substance based on fluorescence from the extinguishable fluorescent substance; and
(d2) extinguishing the extinguishable fluorescent substance that has emitted fluorescence are conducted.

The second biosubstance-binding substance is different from the first biosubstance-binding substance. However, the extinguishable fluorescent substance with which the second biosubstance-binding substance is labeled can be identical to the extinguishable fluorescent substance with which the first biosubstance-binding substance was labeled. Even when the two are identical, since an extinguishing operation has been conducted in (d1), the extinguishable fluorescent substance with which the first biosubstance-binding substance has been labeled does not emit fluorescence when irradiated with the excitation beam of the extinguishable fluorescent substance in (b2), making it possible to determine which biosubstances exhibit binding properties to the second biosubstance-binding substance. Contacting of the second biosubstance-binding substance with the biosubstances, irradiation with an excitation beam, detection of fluorescence, and extinction of the extinguishable fluorescent substance can be conducted in the same manner as in (a1) to (d1). The operations of (a1) to (c1) and (a2) to (c2) make it possible to determine which biosubstances bind the first biosubstance-binding substance and which bind the second biosubstance-binding substance.

In the method of the present invention, following (d2) above, the steps of (a3) bringing a third or subsequent biosubstance-binding substance labeled with an extinguishable fluorescent substance into contact with the multiple immobilized biosubstances following extinction; (b3) irradiating the excitation beam of the extinguishable fluorescent substance; (c3) detecting the biosubstances that have bound to the biosubstance binding-substance labeled with the extinguishable fluorescent substance based on fluorescence from the extinguishable fluorescent substance; and (d3) extinguishing the extinguishable fluorescent substance that has emitted fluorescence, are successively repeated one time each for a third and subsequent biosubstance-binding substances. It is thus possible to evaluate the binding properties of three or more biosubstance-binding substances with the same biosubstances. (a3) to (d3) can be conducted in the same manner as (a1) to (d1).

The third biosubstance-binding substance is different from the second biosubstance-binding substance. However, the extinguishable fluorescent substance with which the third biosubstance-binding substance is labeled may be identical to the extinguishable fluorescent substance with which the second biosubstance-binding substance is labeled. Even when the two are identical, since an extinguishing operation has been conducted in (d2), the extinguishable fluorescent substance with which the second biosubstance-binding substance has been labeled does not emit fluorescence when irradiated with the irradiation of the excitation beam of the extinguishable fluorescent substance in (b3), making it possible to determine which biosubstances exhibit binding properties to the third biosubstance-binding substance. This relation is identical for the third and subsequent biosubstance-binding substances and extinguishable fluorescent substances.

Data relating to the fluorescence that is the result of the evaluation of the binding properties of the multiple biosubstances immobilized on the substrate surface to the biosubstance-binding substance are obtained by the above method. These data can be stored in a computer, for example. Biosubstances of desired binding properties can then be selected from the data that have been stored in the computer.

The biosubstances having desired binding properties that are selected in this manner can be removed from the substrate surface and employed in a subsequent operation. The subsequent operation will vary with the type of biosubstance. For example, when the biosubstance is a B-lymphocyte and the biosubstance-binding substance is antigen, the subsequent operation can be the cloning of an antibody gene from the B-lymphocyte. Alternatively, when the biosubstance is a B-lymphocyte and the biosubstance-binding substance is antigen, the subsequent operation can be the proliferation of antigen-specific B-lymphocytes and the production of hybridomas. When the biosubstance is a T-lymphocyte and the biosubstance-binding substance is an MHC/peptide complex tetramer, the subsequent operation can be the cloning of a T-cell receptor gene from the T-lymphocyte. Alternatively, an antigen-specific T-lymphocyte can be proliferated to establish a cell line. When the biosubstance is a tissue and the biosubstance-binding substance is an autoantibody, the subsequent operation can be to clone an autoantigen gene from the cells in the tissue to which the autoantibody binds. When the biosubstance is cancerous tissue and the biosubstance-binding substance is a cancer antigen-specific antibody, the subsequent operation can be to isolate and analyze cancer cells from cancer tissue.

Further, when the biosubstance is cells and the biosubstance-binding substance is an antibody to a cell surface marker, the method of the present invention can be used to evaluate the binding properties of the cell and the antibody to the cell surface marker. The method of the present invention can be employed to specify types of cells, specify subsets of cells, analyze activated and inactivated states of cells, and analyze the degree of malignancy of cells, such as the cancerous development of cells.

Further, the method of the present invention can be used to provide a method of screening for substances binding specifically to biosubstances. Specifically, in cells in which a biosubstance induces the expression of a cell growth factor receptor, the cells can be successively brought into contact with various fluorescence-labeled substances (candidate substances) to identify molecules binding to the cell growth factor receptor when screening for substances binding to the cell growth factor receptor. Conversely, ligands of fluorescence-labeled receptors can be prepared, and while bringing them into contact with the cells, the effects of various molecules not labeled with fluorescence can be employed to screen for substances that specifically bind to biosubstances and have a blocking effect on the binding of fluorescence-labeled ligands to cells.

### Embodiments

### Embodiment 1

### Fluorescence-extinguishing method

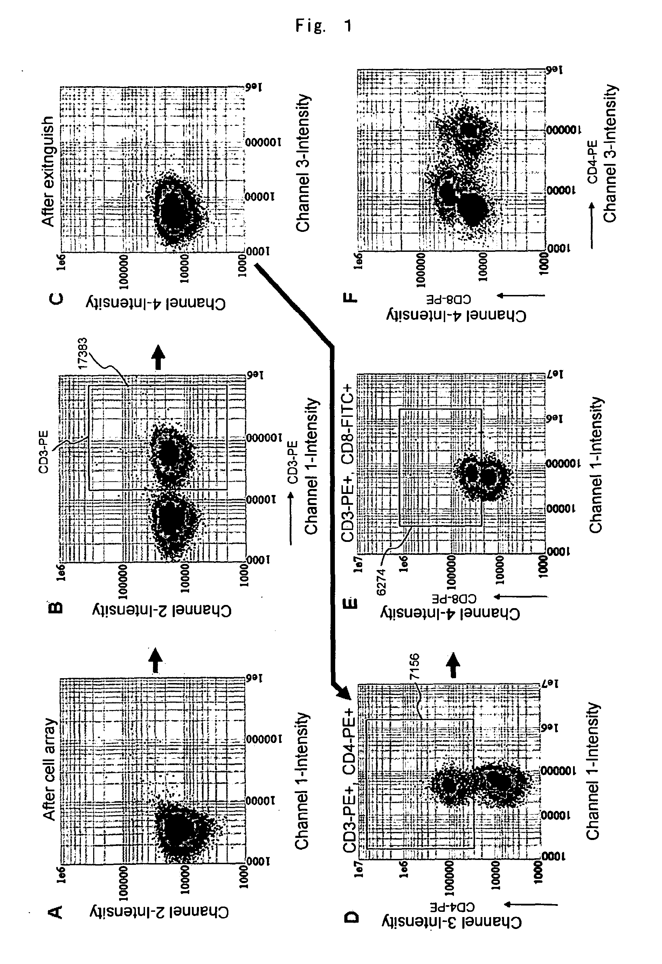

Fig. 1; Multiple staining of T-cells

### 1. Cell preparation

Lymphocytes were obtained by specific gravity centrifugation from human peripheral blood.

### 2. The cell array

1.1 A 50 microliter suspension of 2 x10⁴ human peripheral blood lymphocytes/microliter was added to a microwell array chip on which were regularly arranged 45,000 microwells about 15 micrometers in depth and 10 micrometers in diameter. The cell suspension was left standing for about 2 to 3 minutes and then stirred about three times by pipeting.
1.2 The buffer on the chip was removed by pipet, 50 microliters of buffer was added, and the pipet was used for brief stirring to remove the buffer. This was repeated approximately two more times to remove cells that had not entered wells. Finally, 50 microliters of buffer was added and the cells were covered with a cover glass. The buffer was subsequently replaced about three times.
1.3 The chip was inserted into a fluorescence scanner (CRBIOlle-FITC, Hitachi Software Engineering (Ltd.), Yokohama), scanned on channel 1 (excitation wavelength 532 nm, fluorescence wavelength 585 nm) and channel 2 (excitation wavelength 473 nm, fluorescence wavelength 535 nm) and the fluorescence was measured (Fig. 1A).

### 3. Staining and observation of cells with fluorescent antibody

3.1 With the cover glass still in place, the buffer was removed, 50 microliters of PE-labeled CD3 antibody (Beckman Coulter, Tokyo) diluted 200-fold with PBS was added, and the mixture was left standing at room temperature shielded from light for 5 minutes.
3.2 With the cover glass still in place, the buffer was removed, and 50 microliters of PBS was added. This operation was repeated two more times.
3.3 The chip was inserted in a fluorescence scanner (CRBIOEEe-FITC, Hitachi Software Engineering (Ltd.), Yokohama) and scanned at an excitation wavelength of 532 nm and a fluorescence wavelength of 585 nm to measure the fluorescence (Fig. 1 B).

### 4. Extinction of fluorescence

4.1 The chip was placed under a fluorescence microscope (BX51WI, Olympus) and the fluorescence was extinguished while under observation (approximately several tens of seconds per field of view, approximately several minutes total).
4.2 The chip was inserted into a fluorescence scanner (CRBIOIIe-FITC, Hitachi Software Engineering (Ltd.), Yokohama) and scanned at an excitation wavelength of 532 nm and a fluorescence wavelength of 585 nm to measure the fluorescence and confirm extinction of the fluorescence (Fig. 1 C).

5. Staining and observation of cells with a second type of fluorescent antibody 5.1 With the cover glass still in place, the buffer was removed, 50 microliters of PE-labeled CD4 antibody (IOtest, France) diluted 400-fold with PBS was added, and the mixture was left standing at room temperature shielded from light for 5 minutes.
5.2 With the cover glass still in place, the buffer was removed, and 50 microliters of PBS was added. This operation was repeated two more times.
5.3 The chip was inserted in a fluorescence scanner (CRBIOEEe-FITC, Hitachi Software Engineering (Ltd.), Yokohama) and scanned at an excitation wavelength of 532 nm and a fluorescence wavelength of 585 nm to measure the fluorescence (Fig. 1 D).

### 6. Extinction of fluorescence

6.1 The chip was placed under a fluorescence microscope and the fluorescence was extinguished while under observation (approximately several tens of seconds per field of view, approximately several minutes total).
6.2 The chip was inserted into a fluorescence scanner (CRBIOIIe-FITC, Hitachi Software Engineering (Ltd.), Yokohama) and scanned at an excitation wavelength of 532 nm and a fluorescence wavelength of 585 nm to measure the fluorescence and confirm extinction of the fluorescence.

### 7. Staining and observation of cells with a third type of fluorescent antibody

7.1 With the cover glass still in place, the buffer was removed, 50 microliters of PE-labeled CD8 antibody (IOtest, France) diluted 250-fold with PBS was added, and the mixture was left standing at room temperature shielded from light for 5 minutes.
7.2 With the cover glass still in place, the buffer was removed, and 50 microliters of PBS was added. This operation was repeated two more times.
7.3 The chip was inserted in a fluorescence scanner (CRBIOEEe-FITC, Hitachi Software Engineering (Ltd.), Yokohama) and scanned at an excitation wavelength of 532 nm and a fluorescence wavelength of 585 nm to measure the fluorescence (Fig. 1E).

The results of the fluorescence measurement of Fig. 1 are described in organized fashion below.
A. The results of analysis with a fluorescence scanner of the fluorescence of peripheral blood lymphocytes prior to staining.
B. The results of analysis with a fluorescence scanner of the fluorescence of lymphocytes stained with PE-labeled CD3 antibody. A cell group to which CD3 antibody bound was observed horizontally to the right of the cell group to which CD3 antibody did not bind. That is, T-cells were observed.
C. The results of extinguishing the PE fluorescence of the above cells and observing them with a scanner. Extinction of PE fluorescence was observed.
D. The results of analysis with a scanner of the fluorescence after staining the lymphocytes using PE-labeled CD4 antibody on the cells of C above. A cell group to which CD4 antibody bound was observed above the cell group to which CD4 did not bind.
E. The results of analysis with a fluorescence scanner after extinguishing the PE fluorescence of the cells of D above and then staining the lymphocytes with PE-labeled CD8 antibody. A cell group to which CD8 antibody bound was observed above the cell group to which CD8 antibody did not bind.
F. The results of the analysis of D and E shown simultaneously. Since the position (address) of the cells in the array is fixed, the measurement results for each cell in D and E are displayed with the individual cells superposed. Cell groups of CD4-positive T-cells and CD8-positive T-cells in peripheral blood lymphocytes are shown.

The extinction of fluorescence, the staining of cells with PE-labeled target antibody, and the measurement of fluorescence can be repeatedly conducted. Dilution of the antibody can be suitably adjusted. Determination after titration is desirable. Since the degree of isolation of negative signals and positive signals drops slightly when analyzed by scanner relative to a flow cytometer, this fact is desirably taken into account in advance when determining the antibody dilution rate.

### Embodiment 2

### Fluorescence-extinguishing method

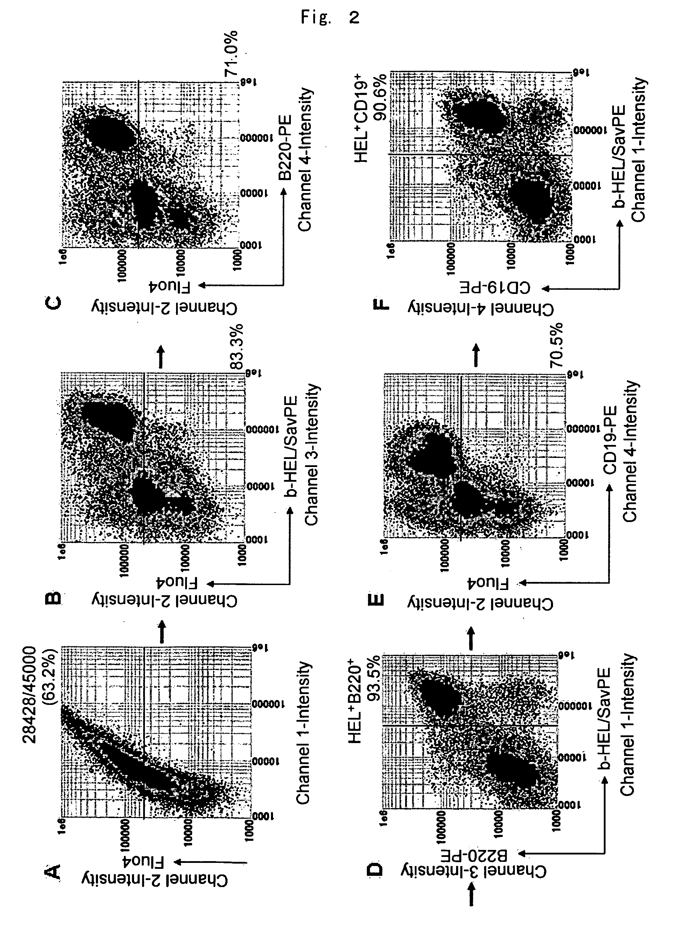

Fig. 2; Multiple staining of B-lymphocytes (B-cells)

### 1. Cell preparation

The spleen of a transgenic mouse (HyHEL10-tg mouse) into which had been introduced the gene for an antibody (HyHEL10) binding to hen egg lysozyme (HEL) was mashed in phosphate buffered saline (PBS) to prepare spleen cells. The cell surface of most of the B-cells of the HyHEL10-tg mouse expressed HEL-specific antibody. The spleen cells were comprised of about 50 percent B-cells.

### 2. The introduction of Fluo-4 into the cells

The spleen cells were mixed with 1 µM of Fluo-4-Am (Molecular Probes, the Netherlands) and 1 µM of Pluronic F127 (Molecular Probes, USA) in loading buffer (PBS, HEPES 20 mM pH 7.6, NaCl 137 mM, KCI 2.7 mM, CaCl₂/2H₂O 1.8 mM, MgCl₂ 0.1 mM, glucose 1.1 mM, BSA 0.2 percent) and then shaken at room temperature for 30 minutes while being shielded from light. Subsequently, the cells were washed three times with loading buffer to remove the Fluo-4-Am that had not been loaded into cells, and the loading buffer was replaced with 10 percent FCS/RPMI solution. The Fluo-4 was used to determine whether or not cells had entered the microwells when reading the chip with a scanner.

### 3. The cell array

1.1 A 50 microliter suspension of 2x10⁴ HyHEL10-tg mouse spleen cells/microliter was added to a microwell array chip on which were regularly arranged 45,000 microwells about 15 micrometers in depth and 10 micrometers in diameter. The suspension was left standing for about 2 to 3 minutes and then stirred about three times by pipeting.
1.2 The buffer on the chip was removed with a pipet, 50 microliters of buffer was added, and the pipet was used for brief stirring to remove the buffer. This was repeated approximately two more times to remove cells that had not entered wells. Finally, 50 microliters of buffer was added and the cells were covered with a cover glass. The buffer was subsequently replaced about three times.
1.3 The chip was inserted into a fluorescence scanner (CRBIOIIe-FITC, Hitachi Software Engineering (Ltd.), Yokohama), scanned on channel 1 (excitation wavelength 532 nm, fluorescence wavelength 585 nm) and channel 2 (excitation wavelength 473 nm, fluorescence wavelength 535 nm), and the fluorescence was measured. B-cells labeled with Fluo-4 were detected by this measurement (Fig. 2A).

### 4. Staining and observation of cells with fluorescence-labeled antigen

4.1 With the cover glass still in place, the buffer was removed, 50 microliters of biotin-labeled HEL diluted to 2 micrograms/mL in PBS was added, and the mixture was left standing at room temperature shielded from light for 5 minutes.
4.2 The buffer was removed with the cover glass still in place, after which 50 microliters of PBS was added. This operation was repeated two more times.
4.3 Next, the buffer was removed with the cover glass still in place, 50 mL of PE-labeled streptavidin (BD Pharmingen, USA) diluted 1,500 fold with PBS was added, and the mixture was left standing at room temperature shielded from light for 5 minutes.
4.4 With the cover glass still in place, the buffer was removed and 50 microliters of PBS was added. This operation was repeated two more times.
4.5 The chip was inserted into a fluorescence scanner (CRBIOlle-FITC, Hitachi Software Engineering (Ltd.), Yokohama), scanned on channel 1 (excitation wavelength 532 nm, fluorescence wavelength 585 nm) and the fluorescence was measured. HEL-specific B-cells were detected by this measurement (Fig. 2B).

### 5. Extinction of fluorescence

5.1 The chip was placed under a fluorescence microscope (BX51WI, Olympus) and the fluorescence was extinguished while under observation (approximately several tens of seconds per field of view, approximately several minutes total).
5.2 The chip was inserted into a fluorescence scanner (CRBIOIIe-FITC, Hitachi Software Engineering (Ltd.), Yokohama) and scanned on channel 1 (excitation wavelength of 532 nm and a fluorescence wavelength of 585 nm) to measure the fluorescence and confirm extinction of the fluorescence.

### 6. Cell staining and observation with fluorescent antibody

By repeating cell staining with a fluorescent antibody and extinction in the same manner, the expression of B220 (Figs. 2C and D) and CD19 (Figs. 2E and F) by mouse B-cells was measured.

The results of Fig. 2 will be described in organized fashion below.
A. The results of analysis with a fluorescence scanner of the spleen cells of an HyHEL10-tg mouse labeled with Fluo-4. Fluo-4 positive cells were observed above the negative cells. The boundary between the Fluo-4 positive cells and negative cells was unclear.
B. The results of analysis by fluorescence scanning of the above cells stained with biotin-labeled HEL and PE-labeled streptavidin. The vertical axis shows Fluo-4 fluorescence and the horizontal axis shows biotin-labeled HEL and PE-labeled streptavidin cell fluorescence. B-cells that expressed HEL antibody on the cell surface and that bound to HEL were observed diagonally upward and to the right.
C. The results of analysis by fluorescence scanning after extinguishing the PE fluorescence of the above cells and using PE-labeled B220 antibody to stain lymphocytes. The vertical axis shows Fluo-4 fluorescence and the horizontal axis shows the fluorescence of cells to which PE-labeled B220 antibody bound. B-cells to which B220 antibody bound were observed diagonally upward and to the right.
D. The results of analysis in B and C. Since the position (address) of the cells in the cell array is fixed, the measurement results for each cell in B and C are displayed with the individual cells superposed. The horizontal axis shows the fluorescence of cells to which biotin-labeled HEL and PE-labeled streptavidin bound, and the vertical axis shows the fluorescence of cells stained by PE-labeled B220 antibody. The B220 antibody is a murine B-cell marker; almost all B220-positive cells were bound by HEL (cell group diagonally upward and to the right). A B220-negative cell group, some of which bound to HEL, was observed.
E. The results of analysis by fluorescence scanner after extinguishing the PE fluorescence of the cells in D above and staining the lymphocytes with PE-labeled CD19 antibody. The vertical axis shows the Fluo-4 fluorescence of the cells and the horizontal axis shows the fluorescence of cells to which PE-labeled CD19 antibody bound. CD19-positive B-cells were observed diagonally upward and to the right.
F. The results of the analysis of B and E. Since the position (address) of the cells in the cell array is fixed, the measurement results for each cell in B and E are displayed with the individual cells superposed. The horizontal axis denotes the fluorescence of cells that were bound by biotin-labeled HEL and PE-labeled streptavidin, and the vertical axis shows the fluorescence of cells stained with PE-labeled CD19 antibody. CD19 is a murine B-cell marker, and almost all of the CD-19 positive cells were bound by HEL (cell group diagonally upward and to the right). A CD19-negative cell group, some of which bound to HEL, was observed.

### Embodiment 3

### Fluorescence-extinguishing method

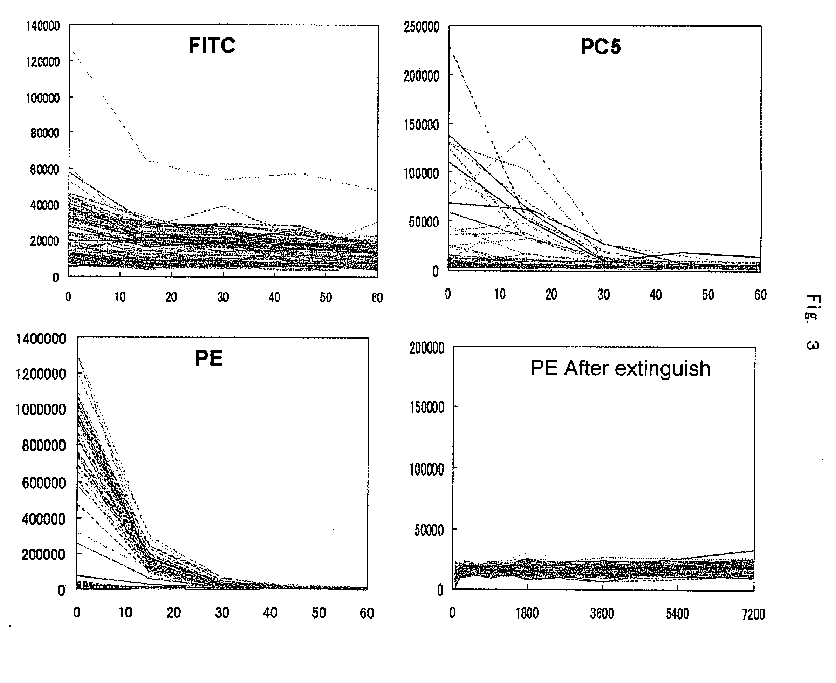

Fig. 3; Extinction after staining of spleen cells

Biotinylated anti-murine CD4 antibody was added to murine spleen cells, the mixture was reacted for 15 minutes at 4°C, and the cells were washed three times with buffer to conduct labeling. Subsequently, equal quantities were charged to test tubes, solutions of streptavidin-labeled PE, FITC, and PC5 were added, the mixtures were reacted for 15 minutes at 4°C, and the reaction products were washed three times with buffer. Titration was conducted for each of the fluorescence pigments and suitable concentrations were employed. The spleen cells that had been labeled with the various fluorescences were arrayed on cell chips (45,000 wells), the fluorescence intensity was scanned, and one cluster (900 wells) was analyzed (at 0 seconds). Subsequently, the same cluster was observed under fluorescent light at 15 second intervals under a fluorescence microscope, after which the operations of scanning with a scanner and analysis were repeated. Groups that did not change at a fluorescence intensity of 5000 or lower indicated cells that were not fluorescence labeled or wells in which cells were not arrayed.

Fig. 3 shows the extinction of the various fluorescent pigments. The vertical axis shows fluorescent intensity, and the horizontal axis shows the time (seconds) from the start of irradiation with fluorescence.

As a result, PE afforded more intense and uniform fluorescence than the other fluorescent pigments, and exhibited rapid and uniform extinction. PC5 yielded extinction results that were nearly equivalent to those of PE, but cell detection was somewhat nonuniform. Extinction, albeit gradual, was confirmed for FITC, but more than 60 seconds was required for complete extinction. In the extinction results for PE, no recovery of fluorescence was observed during a two-hour (7,200 second) observation period. As a result, it will be understood that both PC5 and FITC can be employed in the method of the present invention in almost equivalent fashion to PE.

### Industrial Applicability

The present invention is useful in evaluating the binding properties of biosubstances and biosubstance-binding substances in various fields relating to biosubstances, such as cells.

### Brief Description of the Drawings

[Fig. 1] The results of multiple staining of human T-cells in Embodiment 1 (T-cell multiple staining/human PBMC/no loading/silicon chip (45,000 well)).
[Fig. 2] The results of multiple staining of murine B-cells in Embodiment 2 (B-cell multiple staining/HEL-Tg mouse spleen/Fluo-4 loading/silicon chip (45,000 wells)).
[Fig. 3] The results of multiple staining of murine spleen cells in Embodiment 3.

## Claims

1. A method of evaluating binding properties of multiple biosubstance-binding substances having binding properties to multiple biosubstance against multiple biosubstances immobilized on a substrate surface, comprising:
(a1) bringing a first biosubstance-binding substance labeled with an extinguishable fluorescent substance into contact with said multiple immobilized biosubstances;
(b1) irradiating said multiple immobilized biosubstances with the excitation beam of the extinguishable fluorescent substance;
(c1) detecting the biosubstances that have bound to said biosubstance binding-substance labeled with said extinguishable fluorescent substance based on the fluorescence emitted by said extinguishable fluorescent substance due to said excitation beam,
(d1) extinguishing the extinguishable fluorescent substance that has emitted the fluorescence; then
(a2) bringing a second biosubstance-binding substance labeled with an extinguishable fluorescent substance into contact with said multiple immobilized biosubstances following extinction; thereafter
(b2) irradiating the excitation beam of said extinguishable fluorescent substance;
(c2) detecting the biosubstances that have bound to said biosubstance binding-substance labeled with said extinguishable fluorescent substance based on the fluorescence emitted by said extinguishable fluorescent substance; and
(d2) extinguishing said extinguishable fluorescent substance that has emitted fluorescence.

2. The method in accordance with claim 1, wherein following (d2), the steps of (a3) bringing a third or subsequent biosubstance-binding substance labeled with an extinguishable fluorescent substance into contact with said multiple immobilized biosubstances following extinction; (b3) irradiating the excitation beam of said extinguishable fluorescent substance; (c3) detecting the biosubstances that have bound to said biosubstance binding-substance labeled with said extinguishable fluorescent substance based on fluorescence from said extinguishable fluorescent substance; and (d3) extinguishing said extinguishable fluorescent substance that has emitted the fluorescence, are successively repeated one time each for a third and subsequent biosubstance-binding substances.

3. The method in accordance with claim 1 or 2, wherein said biosubstance is one or more substances selected from the group consisting of cells, tissues, viruses, bacteria, proteins, nucleic acids, lipids, sugar chains, hormones, and products thereof.

4. The method in accordance with any one of claims 1 to 3, wherein said biosubstance-binding substance is one or more members selected from the group consisting of antibodies, antigens, ligands, hormones, proteins, nucleic acid, lipids, and sugar chains.

5. The method in accordance with any one of claims 1 to 4, wherein said extinguishable fluorescent substance is phycoerythrin (PE), fluorescein, or allophycocyanine (APC).

6. The method in accordance with any one of claims 1 to 5, wherein the florescence of said extinguishable fluorescent substance is extinguished by means of irradiation by a mercury lamp or a halogen lamp.

7. The method in accordance with any one of claims 1 to 6, wherein said excitation beam of said extinguishable fluorescent substance is a monochromatic beam.

8. The method in accordance with claim 7, wherein said monochromatic beam is a laser beam.

9. The method in accordance with any one of claims 1 to 8, wherein data relating to fluorescence in the form of the evaluation results of the binding properties of the biosubstance-binding substances to the multiple substances immobilized on the substrate surface are stored in a computer and a biosubstance having desired binding properties is selected from the stored data.

10. The method in accordance with claim 9, wherein the biosubstance that is selected is removed from the substrate surface and employed in a subsequent operation.

11. The method in accordance with claim 10, wherein said biosubstances are cells and said method is employed to evaluate the binding properties of cells and cell surface markers.

12. A method of screening for substances having specific binding properties to biosubstances by employing the method in accordance with any one of claims 1 to 11.
